# EUROPEAN PATENT SPECIFICATION

(11) **EP 2 182 931 B1**
(45) Date of publication and mention of the grant of the patent: **20.04.2016**
(21) Application number: 08786280.1
(22) Date of filing: 21.07.2008
(51) Int. Cl.: A61K 9/70, A61K 47/38

(54) **DERIVATISED POLYSACCHARIDE MATERIAL FOR THE TOPIC ANTIBACTERIAL ACTIVITY**
DERIVATISIERTES POLYSACCHARID-MATERIAL FÜR TOPISCHE ANTIBAKTERIELLE AKTIVITÄT
MATIÈRE POLYSACCHARIDIQUE TRANSFORMÉE EN DÉRIVÉ POUR L'ACTIVITÉ ANTIBACTÉRIENNE TOPIQUE

(30) Priority: 26.07.2007 WO PCT/IT2007/000530
(43) Date of publication of application: 12.05.2010
(73) Proprietor: Humanitas Mirasole S.p.A., 20089 Rozzano (Milano) (IT); Politecnico di Milano, 20133 Milano (IT); Istituto di Ricerche Chimiche e Biochimiche Giuliana Ronzoni, 20133 Milano (IT)
(72) Inventor: GRAZIANI, Giorgio, 20089 Rozzano Milano (IT); MONTANELLI, Alessandro, 20089 Rozzano Milano (IT); MELONE, Lucio, I-20133 Milano (IT); VISMARA, Elena, I-20133 Milano (IT); TORRI, Giangiacomo, I-20133 Milano (IT)
(74) Representative: Long, Giorgio
(86) International application number: PCT/EP2008/059509
(87) International publication number: WO 2009/013261

(56) References cited:
- WO-A-01/48025
- DE-A1- 4 035 378
- US-A- 3 420 788
- US-A- 4 357 468
- US-A- 5 854 146
- US-A- 6 048 736

## Description

### Field of the invention

The present invention regards preferably bacteriostatic or bactericidic antibiotics linked to polysaccharide materials.

### Background art

The most common drug administration path is the oral administration, in which the active principle is normally absorbed through the intestine, metabolised by the liver, released in the blood flow and then distributed throughout the organism.

In most cases, the liver metabolises the active principle in order to facilitate its elimination, i.e. it renders it more water soluble so it can be expelled through the urine or faeces. The orally administered drug should have an adequate dosage to achieve an effective blood concentration, taking into account the partial liver metabolism.

Other drug administration paths, while not being as simple or immediate as the oral path, can however be very useful in particular pathological conditions. For example, in patients with severe burns over most of the body, the skin is the most affected organ. Skin is the natural barrier protecting the organism from external bacterial penetration. In patients with extensive burns, this natural protection is lacking, leading to great danger of bacterial infection with high risk of sepsis. In fact it is known that the majority of burned patients, being the most of their body destroyed, are at high risk of sepsis. In fact, it is not so much by damage from the burning, but by serious bacterial infections occurring during the period following their hospital recovery.

In these cases, the oral administration of antibiotics is often not sufficient to prevent bacterial-induced infections. In these patients the antibacterial action should directly derive by the contact or antibacterial topic action of textile tissues that act preventing the bacterial penetration into the blood flow.

By means of a combined oral and topical administration of the antibacterial drug, one can one hope to reduce patients mortality due to post-burn sepsis.

The topical antibacterial and antimicotic action of the drug should obtain an optimal protection from the possible penetration of harmful external agents.

### Summary of the invention

The problem underlying the present invention is therefore that of making available a device which, in a constant manner when placed in contact with the skin or a skin wound, acts as topic antibacterial agent.

Such problem is resolved by a textile material as outlined in the attached claims.

The present invention then regards a textile material, preferably of polysaccharide nature or generally having an hydrocarbon moiety, derivatised with an appropriate monomer of synthetic nature, loaded/saturated with a pharmaceutical active principle.

In another aspect, the invention regards a method for preparing such textile material and its use.

### Brief description of the drawings

Figure 1 shows a graph of affinity drug-gauzes versus contact time;
Figure 2 shows a graph of affinity drug-gauzes versus contact time, at a different drug concentration with respect to figure 1;
Figure 3 shows an adsorption isotherm drug-gauzes at different drug concentrations.

### Detailed description of the invention

It was surprisingly found that hydrocarbon-carrying substrates and in particular polysaccharide substrates derivatised with monomers are capable of absorbing different types of drugs acting as antibacterial agents in contact with the skin, even in the absence of cyclodextrins. A material thus functionalised can be employed for the topic skin bacterial protection.

The term "hydrocarbon-carrying substrate" indicates a substrate that is made of an hydrocarbon chain or ring or an oligomer or a polymer thereof or any other substrate that derives from it or in general a substrate that contains a hydrocarbon moiety.

The monomers preferably used in the present invention are chosen from among glycidyl methacrylate (GMA) or derivatives thereof, acrylic acid o derivatives thereof, N-vinylpyrrolidone, acrylamide or derivatives thereof and vinyl acetate. Monomers of acrylic nature are preferably employed. More preferably, use is made of glycidyl methacrylate and its diol derivative.

The polysaccharide material advantageously used in the present invention is chosen from among a fabric or non-woven fabric made of cellulose or cellulose derivative, such as cellulose acetate. The preferred substrate is cellulose fabric, for example cotton or linen.

Other preferred hydrocarbon-carrying substrates that can be used for the purposes of the present invention are selected from polyamides, polyesters, polypropylene or other polyolefins or silk.

The method for functionalising the polysaccharide substrate of the present invention is described in the scientific publication Vismara et al., European Polymer Journal 41 (2005), 1787-1797 and in the European patent No. 0 242 172.

The polysaccharide substrate is treated with both physical and chemical free radical sources (physical sources such as irradiation by means of e-beam or chemical sources such as the Fenton's reagent), causing the formation of stable carbon radicals over time.

The radicals thus formed on the polysaccharide or hydrocarbon moiety are quenched by making the substrate to react with a monomer chosen from among glycidyl methacrylate (GMA) or derivatives thereof, acrylic acid o derivatives thereof, N-vinylpyrrolidone, acrylamide or derivatives thereof and vinyl acetate.

If glycidyl methacrylate is used as a monomer, a glycidylcarbonyl-2-propyl functionalised substrate is formed. The epoxy ring can be subsequently opened, in strongly aqueous ionic conditions, to give the corresponding diol monomer, i.e. a (3-glycerol-O-carbonyl)-2-propyl functionalised substrate.

The substrate thus functionalised is employed as such. The polysaccharide substrate derivatised with the above-described monomers is immersed in an aqueous solution of an active principle, typically a drug, and subjected to stirring for a time in the range of 1 - 96 hours, preferably for about 72 hours. The active principle is then absorbed by the substrate and retained inside the substrate itself. Once the stirring is terminated, the textile material is washed with deionised water and then dried in a manner such as to eliminate the excess non-absorbed solution. The aqueous solution employed for the impregnation can contain the active principle as such or formulated with pharmaceutically acceptable excipients which, therefore, will be absorbed on the substrate together with the active principle.

By measuring the weight of the textile material before and after the loading of the drug, the quantity of absorbed active principle is determined.

The polysaccharide or hydrocarbon-carrying substrate derivatised with the above described monomers and loaded with one or more active principles is then applied on the affected zone, i.e. on the skin or on a wound for times which vary according to the type of drug and the type of treatment to be carried out. The drug, in contact with the skin or a wound, acts as a topic antibacterial agent in a continuous manner.

Examples of drugs or active principles acting as topic agents with the device of the present invention include: anti-inflammatories, sedatives, hypnotics, analgesics, anaesthetics, antibiotics, drugs for heart pathologies, antipyretics, antidepressives and antispastics

The preferred active principles acting as topic agents of the present invention are antibiotics. Examples of antibiotics include: penicillin, aminoglycosides, quinolone antibiotics, tetracycline, chloramphenicol, sulfacetamide, sulfamethazine, sulfadiazine, sulfamerazine, sulfamethizole, erythromycin, clarythromycin, rifamycin, rifampycin, ceftazidime, amoxicillin, amoxicillin trihydrate, clavulanic acid, vancomycin chlorohydrate, teicoplanin, linezolid. The antibiotics preferably employed are anti-staphylococcal and anti-funginal such as rifamycin sodium salt, ceftazidime pentahydrate, amoxicillin trihydrate and potassium clavulanate, vancomycin chlorohydrate, teicoplanin and linezolid, possibly formulated with pharmaceutically acceptable excipients.

The amount of drug to be incorporated in the material of the invention varies with the type of active principle and the desired therapeutic effect. Normally, for the objects of the invention the quantity of drug absorbed on the cellulose or hydrocarbon-carrying substrate varies from 0.0001% to 60%.

The textile material of the present invention is preferably employed for antibacterial and antimicotic antibiotics. In a preferred embodiment, the textile material is a linen item, such as a sheet impregnated with one or more antibiotics, the said sheet being used in particular for covering the patients which have extensive skin burns, multi-traumatized patients, marrow transplanted patients, long-term patients with decubitus ulcers, diabetic foot infections, premature infants or patients with serious dermatitis, in order to protect them from possible bacterial infections (typically, by Gram+ bacteria). Other linen items can be a pillowcase, a towel or the like.

According to such embodiment, the inventive textile material can also be a bandage or a gauze to cover the patient skin that has been offended by the burns. According to an other embodiment, the textile material is a gauze pad of the type used in surgery, particularly the gauze pads that are positioned on the edges of the incision that are maintained open by the retractor means. This is in fact a site that is subject to infection, typically by Gram- bacteria, particularly in case of abdomen, vascular, urological surgery and in organ transplants. The use of sterile tools as well as a sterile environment is not however sufficient, in some cases, to avoid developing infection, due to possible contamination by the patient himself/herself. The medicated gauze pads of the invention can thus help in overcoming this problem.

According to another embodiment of the invention, the textile material is a suture, for both cutaneous and visceral use. In fact, in both cases the risk of bacterial infection is quite high, particularly by Gram+ (cutaneous application) and Gram- (visceral application) bacteria.

The material of the present invention is very versatile, since it is capable of absorbing drugs which are even quite structurally diverse, such as clavulanic acid and vancomycin chlorohydrate which have molecular weights of 199.16 and 1485.71, respectively.

Moreover, the polysaccharide material functionalised with the monomers of the invention has shown to be active in absorbing the drug even in the absence of additional functionalisation with cyclodextrins. This allows obtaining a material capable of absorbing drugs which is more economical than the known materials functionalised with cyclodextrins.

### EXPERIMENTAL PART

### FUNCTIONALISATION OF CELLULOSE BY MEANS OF ACRYLATES.

The process of functionalisation of polysaccharides through free radical sources, both physical like irradiation by means of e-beam and chemical like the Fenton's reagent, has already been the subject of studies whose results are reported both in scientific publications (Vismara et al., European Polymer Journal 41 (2005) 1787-1797) and patents (Vismara et al., 2002, European Patent application EP02425172). Diagram 1 shows the mechanisms of activation of cellulose substrates through e-beam or Fenton's reagent due to the non-selective formation of stable carbon radicals over time. Diagram 2, on the other hand, illustrates the quenching process of the radicals thus formed on the polysaccharide, using glycidyl methacrylate. All experimental parameters related to the processes described in Diagrams 1-2 are reported in the previously mentioned literature.

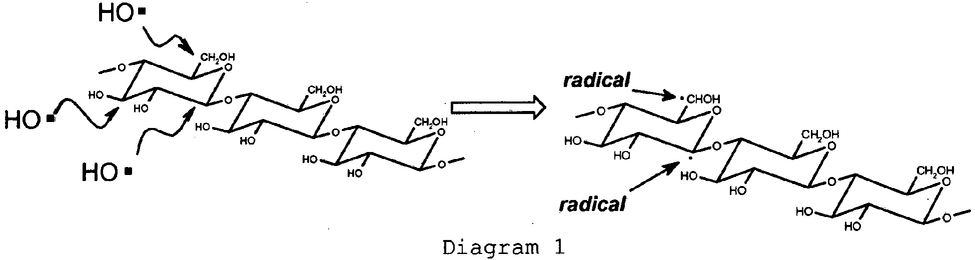

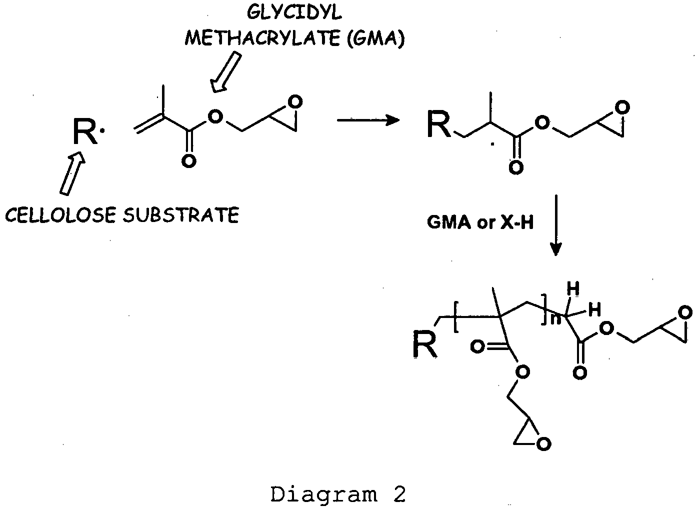

It should be noted that, from the structural point of view, the functionalisation of a polysaccharide or hydrocarbon-carrying substrate by using Fenton reagent or Electron Beam (EB) technology brings to different results. In fact, the Fenton reagent causes a radical formation which involves the bulk structure, while EB acts only on the surface of the substrate. This means that, depending on the technology used, the monomer linked to the substrate will be differently exposed to the exterior. In the case of radical formation by Fenton reagent, the functionalised substrate can thus provide for a prolonged topic antibacterial function.

If one uses glycidyl methacrylate as monomer, the epoxy ring can be conveniently opened, in strongly ionic aqueous conditions, to give the corresponding diol monomer according to Diagram 3 reported below:

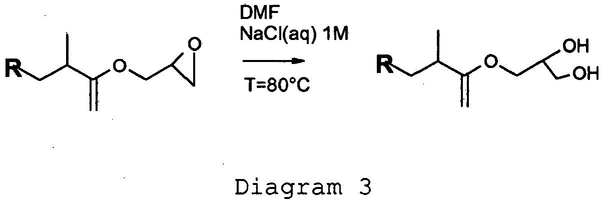

### EXAMPLE - OPENING OF THE EPOXY RING

The opening of the epoxy ring is carried out with the procedure illustrated below. 1 g of material is inserted in a 250 mL 1-neck flask equipped with reflux condenser with 100 mL of anhydrous DMF (dimethylformamide). The system is placed under stirring with magnetic stirrer for 2h at room temperature. Subsequently, the flask is heated in an oil bath up to the temperature of 80°C; once this temperature is reached, 200 mL of 1M NaCl aqueous solution are added. It is left to react at 80°C for 18 h under stirring.

After this time has passed, the flask is allowed to cool, the liquid is decanted, deionised water (about 100 mL) is poured through the reflux and the resulting mixture is magnetically stirred for 30' at 80°C in an oil bath. The operation is repeated 3 times. Subsequently, it is washed 3 times (20' per washing) with 100 mL of acetone. It is left to dry in air until it reaches constant weight.

### DRUG IMPREGNATION OF DIVERSELY FUNCTIONALISED CELLULOSE SUBSTRATES

The material to which GMA was covalently bonded was subjected to drug impregnation.

Both the cellulose substrate as such and that functionalised with GMA (with closed and open ring) were subjected to the same tests in order to compare the complexing properties of molecules of pharmaceutical interest.

### MATERIALS

Cellulose material used for the treatment:
Cotton gauze functionalised with glycidyl methacrylate (GMA) and β-cyclodextrin (TC8) (COMPARATIVE SAMPLE);
Cotton gauze functionalised with GMA (TC3), with open and closed epoxy ring;
Cotton gauze as such (TC0).

Drugs used:
500 mg RIFOCIN (Lepetit Aventis).
Solution ready for intravenous use. 1 10ml vial.

### Composition:

Rifamycin SV sodium salt (Mw=719.8 g/mol) equivalent to 500 mg Rifamycin SV (Mw=698.0 g/mol)
Ascorbic acid;
Edetate disodium;
Sodium metabisulphite;
Propylene glycol;
Sodium Bicarbonate;
Water for injectable preparations.

1g/100mL GLAZIDIM (Glaxosmithkline).

Powder and solvent for infusion solution. 1 powder bottle with Monovial device + 100 mL infusion sack.

### Composition:

1.164 g Ceftazidime pentahydrate (Mw=636.6 g/mol) equivalent to 1g Ceftazidime (Mw=546.6g/mol);
Anhydrous sodium carbonate;
AUGMENTIN, 675mg+125mg (Glaxosmithkline).
Powder for oral suspension.

### Composition:

Amoxicillin trihydrate (Mw=419.5 g/mol) corresponding to 875 mg amoxicillin (Mw=365.4 g/mol);
Potassium clavulanate corresponding to 125 mg clavulanic acid (Mw=199.2 g/mol);
Crospovidone;
Hydrated colloidal silica;
Aspartame;
Magnesium stearate;
Fish aroma - lemon aroma - strawberry aroma;
500 mg COPOVAN (Mayne Pharma srl)
Powder for injectable solution for intravenous and oral use.

### Composition:

512.57 mg Vancomycin chlorohydrate equivalent to 500 mg Vancomycin.

### GAUZE TREATMENT PROCEDURE

SOLUTION A: The 500 mg RIFOCIN of the small 10 ml vial was diluted with 20 mL deionised water in a 100 mL flask. The Rifamycin concentration in the solution thus made is equal to 2.39E-2 M.

SOLUTION B: The powder contained in the GLAZIDIM bottle was weighed: 1265.0 mg.

Since every bottle contains 1164 mg of Ceftazidime pentahydrate, by subtraction there are 101 mg of Na₂CO₃ (7.98%w). Wishing to make an aqueous solution of GLAZIDIM which has the same concentration as the Rifamycin in SOLUTION A (2.39E-2 M), 495.5 mg of GLAZIDIM were weighed, which contain 456.0 mg (7.16E-4 mol) of Ceftazidime pentahydrate, and then dissolved in 30 mL of water in a 100 mL flask.

SOLUTION C: A packet of AUGMENTIN contains 875 mg of Amoxicillin. Since one packet contains 1431.0 mg, 61.1% is composed of Amoxicillin. Wishing to make an aqueous solution of AUGMENTIN which has the same concentration as Rifamycin in SOLUTION A (2.39E-2M), 428.4 mg of AUGMENTIN were weighed, which contain 261.8 mg (7.17 mol) of Amoxicillin, and then dissolved in 30 mL of water in a 100 mL flask.

SOLUTION D: The contents of a small bottle of COPOVAN (512.57 mg, 3.537E-4 mol) are dissolved in 30 mL of deionised water (1.179E-2 M), using a 100 mL flask. GAUZES: the following gauze samples were prepared: TC8 (COMPARATIVE SAMPLE), closed ring TC3, open ring TC3, and TC0 by cutting from the original 2cm x 2cm squares. Before being weighed, the gauzes were dried in a heater at 55°C for 1h.

The gauzes were subsequently inserted in the flasks corresponding to the four solutions A, B, C and D. The latter were placed under stirring on a shaker JULABO SW22 at 100 rpm for 72 h at room temperature.

Once drawn from the respective solutions, the gauzes were dried on filter paper, washed with about 200 mL of cold deionised water (T=5-10°C) on a sintered glass filtered and dried once again on filter paper. Finally, they are placed in a 100 ml glass flask and held under mains vacuum for at least 24 h.

It is appropriate to wash well the gauzes treated with AUGMENTIN, since the drug forms a suspension in water. Moreover, the need to centrifuge the gauzes to eliminate excess water was not shown, since the above-described treatment seems more than sufficient to obtain a clean material.

### EVALUATION OF THE ANTIBACTERIAL ACTIVITY EXPRESSED BY TEXTILE MATERIAL SAMPLES

The samples were processed in a blind study, by means of a lab procedure referable to that which is prepared in the microbiology sections of clinical analysis laboratories for the evaluation of the antibiotic sensitivity/resistance of bacterial agents to antibiotics (Kirby-Bauer method).

The KB method provides for:
- spreading a culture broth, with the presence of bacteria in standardised concentration, on Petri dishes with culture medium constituted by agar added with substances favouring bacterial growth (Mueller-Hinton medium);
- depositing, on the spread medium, a disc of absorbent paper impregnated with an antibiotic;
- observing the presence/absence of bacterial growth inhibition halos at the disks with antibiotic substance and related measuring of the same;
- detecting the presence of a bacterial growth inhibition halo (with the expected size) permits judging the antibiotic sensitivity of the bacterial agent in culture towards the tested antibiotic.

The following were prepared in the specific case:
- cultures of standardised bacterial strains (0.5 McFarland): Gram+ Staphylococcus aureus ATCC 29213 strain; Gram- Escherichia coli ATCC 25922 strain;
- discoids of the textile material under examination. Then the following procedure was followed:
- spreading selected bacterial strains on Petri culture dishes;
- depositing, on the bacterial strain - spread dishes, discoids of textile material under examination;
- observing the presence/absence of growth inhibition halos;
- measuring the diameter of the observed inhibition halos.

The test was repeated in two separate and distinct sessions on 17/01/2007 and on 19/01/2007.

The obtained results are reported in Table 1.

**Table 1**

| **Date** | | **17/01/2007** | **19/01/2007** | | **17/01/2007** | **19/01/2007** | |
|---|---|---|---|---|---|---|---|
| **Sample** | **Description** | **Strain 1** | **Strain 1** | **Mean** | **Strain 2** | **Strain 2** | **Mean** |
| | | **(cm)** | **(cm)** | | **(cm)** | **(cm)** | |
| **TC0** | Cotton gauze without treatments | NO | NO | | NO | NO | |
| **TC31** | TC0 gauze + AUGMENTIN | NO | NO | | NO | NO | |
| **TC32** | Open ring TC3 gauze + AUGMENTIN | 2,5 | | **2,5** | 2,5 | 2,0 | **2,3** |
| **TC33** | TC0 + GLAZIDIM | NO | NO | | NO | NO | |
| **TC34** | Open ring TC3 + GLAZIDIM | 2,5 | 3,0 | **2,8** | 2,0 | 2,0 | **2,0** |
| **TC38** | Closed ring TC3 gauze + COPOVAN | NO | NO | | 1,5 | 1,5 | **1,5** |
| **TC39** | Open ring TC3 gauze + COPOVAN | NO | NO | | 1,5 | 1,5 | **1,5** |

| | | | | | | | |
|---|---|---|---|---|---|---|---|
| **Strain 1 Escherichia coli ATCC 25922** **Strain 2 Staphylococcus aureus ATCC 29213** | | | | | | | |

### Observations.

TC11 is a TC8 gauze + Glazidim (COMPARATIVE SAMPLE);
TC9 is a TC8 gauze + Rifamycin (COMPARATIVE SAMPLE).
TC0, TC8, TC31, TC33: no evidence of antibacterial activity
TC32, TC34, TC40: evidence of antibacterial activity towards Gram + and towards Gram-
TC38, TC39: evidence of antibacterial activity only towards Gram+
TC36 (COMPARATIVE SAMPLE): evidence of antibacterial activity towards Gram+ and Gram-, but with reduction of the inhibition halo (decline of the activity)
TC37 (COMPARATIVE SAMPLE): evidence of antibacterial activity towards Gram+, with increase of the inhibition halo.

The results are consistent with the expected antibiotic sensitivities: E.coli sensitive to Amoxicillin + Clavulanic Acid (AUGMENTIN) and to Ceftazidime (GLAZIDIM); Staphylococcus aureus sensitive to Amoxicillin + Clavulanic Acid (AUGMENTIN), to Ceftazidime (GLAZIDIM), to Rifampycin (RIFADIN) and to Vancomycin (COPOVAN).

In Table 2, the results relate to tests carried out on functionalised cotton gauzes treated with drugs of various types, such tests not reported in the above Table 1.

Unlike that shown in Table 1, the results of Table 2 are only qualitative, where the "YES"es correspond to the presence of bacterial growth inhibition and the "NO"s show the lack of bacterial growth inhibition.

**Table 2**

| **Code** | **Description** | **No.** | **BACTERIAL SUSPENSION (MF)** | | | | | |
|---|---|---|---|---|---|---|---|---|
| | | | **0,5** | | **0,05** | | **0,005** | |
| | | | STAPHYLOCOCCUS AUREUS ATCC29213 | ESCHERICHIA COLI ATCC25922 STRAIN | STAPHYLOCOCCUS AUREUS ATCC29213 | ESCHERICHIA COLI ATCC25922STRAIN | STAPHYLOCOCCUS AUREUS ATCC29213 | ESCHERICHIA COU ATCC25922 STRAIN |
| **TC10** | TC3 gauze + RIFAMYCIN | 1 | **YES** | **NO** | **YES** | **NO** | **YES** | **NO** |
| **TC12** | TC3 gauze + GLAZIDIM | 1 | **NO** | **YES** | **NO** | **YES** | **NO** | **YES** |
| **TC14** | TC3 gauze + AUGMENTIN | 1 | **NO** | **NO** | **NO** | **NO** | **NO** | **NO** |

| | | | | | | | | |
|---|---|---|---|---|---|---|---|---|
| **YES = Presence of inhibition halo; NO = Absence of inhibition halo;** | | | | | | | | |

Finally, in Table 3 the above-reported biological tests are shown, subdivided based on the drug (partially quantitative data).

**Table 3**

| **SUMMARY OF BIOLOGICAL TESTS ON DIVERSELY FUNCTIONALISED COTTON GAUZES** | | | | |
|---|---|---|---|---|
| | | | **Escherichia coli ATCC 25922** | **Staphylococcus Aureus ATCC 29213** |
| | | **AUGMENTIN** | | |
| 1 | **TC31** | TC0 gauze | NO | NO |
| 2 | **TC14** | TC3 (epoxy) gauze | NO | NO |
| 3 | **TC32** | TC3 (open epoxy glycidyl) gauze | 2.5 | 2.3 |
| 4 | **TC13** | TC8 gauze | YES | YES |
| 5 | **TC40** | TC8 gauze | 3.0 | 2.5 |

| | | **GLAZIDIM** | | |
|---|---|---|---|---|
| 1 | **TC33** | TC0 gauze | NO | NO |
| 2 | **TC12** | TC3 (epoxy) gauze | YES | NO |
| 3 | **TC34** | TC3 (open epoxy glycidyl) gauze | 2.8 | 2.0 |
| 4 | **TC11** | TC8 gauze | YES | YES |
| 5 | **TC36** | TC11 release gauze | 1.3 | 0.8 |
| | | | | |

| | | **RIFAMYCIN** | | |
|---|---|---|---|---|
| 1 | | TC0 gauze | Untreated | |
| 2 | **TC10** | TC3 (epoxy) gauze | Ineffective | YES |
| 3 | | TC3 (open epoxy glycidyl) gauze | Untreated | |
| 4 | **TC9** | TC8 gauze | Ineffective | YES |
| 5 | **TC37** | TC9 release gauze | Ineffective | 3.50 |
| | | | | |

| | | **COPOVAN** | | |
|---|---|---|---|---|
| 1 | | TC0 gauze | Untreated | |
| 2 | **TC38** | TC3 (epoxy) gauze | Ineffective | 1.5 |
| 3 | **TC39** | TC3 (open epoxy glycidyl) gauze | Ineffective | 1.5 |
| 4 | | TC8 gauze | Untreated | |

| | | | | |
|---|---|---|---|---|
| **NOTES** **TC 0** Cotton gauze as such, not treated with drugs **TC 3** Cotton gauze functionalised with GMA **TC 8** Cotton gauze functionalised with GMA and β-cyclodextrin (COMP. EX,) | | | | |

### Cultures of standardised strains (0.5 McFarland)

As can be inferred from the results shown in the above-reported tables, cellulose gauzes derivatised with glycidyl methacrylate with open epoxy ring (glycerol derivative) and loaded with antibiotic have an antibacterial activity level comparable to the gauzes functionalised with glycidyl methacrylate and cyclodextrin.

The polysaccharide and hydrocarbon-carrying material of the present invention therefore constitute a valid alternative to the materials functionalised with cyclodextrin since they have the same antibacterial activity but are more economical and easy to prepare as cyclodextrins are not employed.

The functionalization of the substrate with the monomers of the invention is given for each glycosidic ring by the molar substitution (MS) that can range from 0 to 2, preferably from 0.2 to 1. With the term "molar substitution", also shortened to MS, it is intended the average number of (3-glycerol-O-carbonyl)-2-propyl units for a glycosidic unit of substrate, as it is determined by FT-IR spectroscopy and/or by the increase of the weight of the final material. The FT-IR spectroscopy data depend on the fibre surface derivatization while the weight increment is related to the total polymer mass derivatization degree. In this last case, blank control is done on activated but not derivatized samples, but, in the case of gauzes such procedure results low reproducible.

The FT-IR data are obtained by an arbitrary scale as percentage between the GMA ester band at 1726 cm⁻¹ with respect to a cellulose band between 780 and 465 cm⁻¹ on a KBr spectra.

It has also been found that, at a given MS, the reaction kinetic bringing to the link of an active principle to the functionalised substrates of the invention, particularly to the (3-glycerol-O-carbonyl)-2-propyl functionalised substrates, is such as to allow the preparation of charged substrates having predictable doses of active principle bound to the substrate.

The parameters governing this phenomenon are substantially two: i) time of contact of the functionalised substrate with a solution of active principle; ii) concentration of the active principle in the said solution.

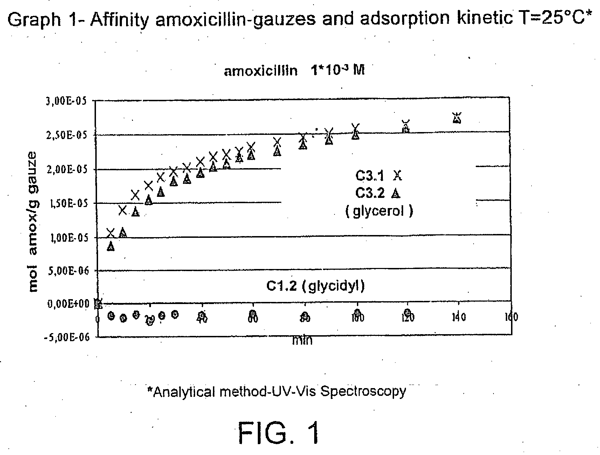

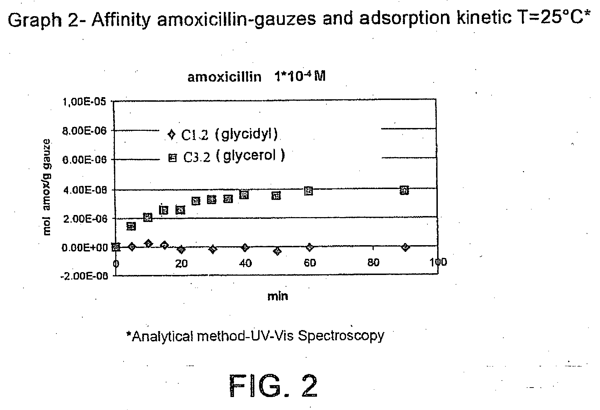

Figure 1 and figure 2 show the adsorption kinetic of amoxicillin (mol amox/g gauze) versus the time of contact, at two different concentration of amoxicillin (10⁻³ M and 10⁻⁴ M, respectively). In both instances, the (3-glycerol-O-carbonyl)-2-propyl functionalised substrate binds the antibiotic molecule in a time depending way, according to a predefined kinetic function.

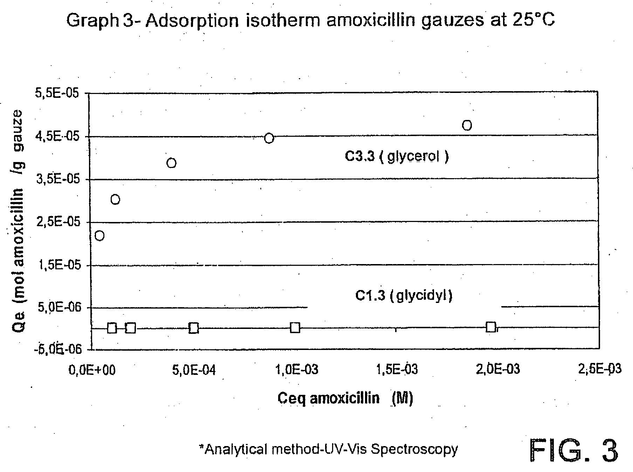

Figure 3 conversely shows the adsorption kinetic of amoxicillin (mol amox/g gauze) versus amoxicillin molar concentration, after a given contact time. Again, the adsorption kinetic of the active principle on the functionalised substrate follows a predefined adsorption function for the glycerol derivative.

The above results are very important, as it possible to prepare a substrate charged with predefined amounts of the active principle, that can vary according to the active principle used, the patient's needs, the kind of use to which the substrate is destined and so on. It is also clear that in such a way the obtainment of the functionalised substrates charged with the active principle is reproducible, giving a greater safety and efficacy in its use.

## Claims

1. Polysaccharide or hydrocarbon-carrying material functionalised with a monomer chosen from the group constituted by glycidyl methacrylate or the glycerol derivative thereof, acrylic acid, N-vinylpyrrolidone, acrylamide and vinyl acetate, **characterised in that** the said material is impregnated with at least one pharmaceutical active principle and that cyclodextrins are absent.

2. Material according to claim 1, which is chosen among a cellulose fabric, a cellulose non-woven fabric, polyamides, polyesters, polypropylene or other polyolefins or silk.

3. Material according to claim 2, wherein, when it is a cellulose fabric or a cellulose non-woven fabric, it is linen or cotton.

4. Material according to any one of the claims 1-3, which is a linen or cotton sheet.

5. Material according to any one of the claims 1-3, which is a linen item, a pillowcase, a bandage, a gauze, a gauze pad for surgery or a suture.

6. Material according to any one of the claims 1 - 5, wherein said monomer is a diol derivative of glycidyl methacrylate.

7. Material according to any one of claims 1-6, which is a (3-glycerol-O-carbonyl)-2-propyl functionalised substrate.

8. Material according to any one of the claims 1 - 7, wherein said at least one active principle is chosen from the group constituted by anti-inflammatory drugs, sedatives, hypnotics, analgesics, anaesthetics, antibiotics, drugs for heart pathologies, antipyretics, antidepressives, antispastics.

9. Material according to any one of the claims 1 - 8 wherein said at least one active principle is at least one antibiotic chosen from the group constituted by penicillin, aminoglycosides, quinolone antibiotics, tetracycline, chloramphenicol, sulfacetamide, sulfamethazine, sulfadiazine, sulfamerazine, sulfamethizole, erythromycin, clarythromycin, rifamycin, rifampycin, ceftazidime, amoxicillin, amoxicillin trihydrate, clavulanic acid, vancomycin chlorohydrate, teicoplanine, linezolid.

10. Material according to claim 9, wherein said at least one antibiotic is chosen from the group constituted by rifamycin sodium salt, ceftazidime pentahydrate, amoxicillin trihydrate and potassium clavulanate, vancomycin chlorohydrate.

11. Material according to any one of the claims 1 - 10, wherein said at least one active principle is possibly formulated with pharmaceutically acceptable excipients.

12. Material according to any one of the claims 1 - 11, wherein the quantity of said at least one active principle absorbed on the cellulose substrate varies from 0.0001% - 60%.

13. Method for preparing the material according to any one of the claims 1 - 12 comprising the steps of:
a) determining the formation of stable free radicals by means of chemical or physical treatment of a cellulose substrate;
b) making the free radicals thus formed to react with a monomer chosen from among the group constituted by glycidyl methacrylate, acrylic acid, N-vinylpyrrolidone, acrylamide and vinyl acetate;
c) if the monomer is glycidyl methacrylate, optionally treating the substrate functionalised according to step b) in strongly ionic aqueous conditions, so as to cause the opening of the epoxy ring, to give the corresponding diol derivative;
d) immersing the derivatised substrate in an aqueous solution of at least one active principle for a time in the range of 1 - 96 hours.

14. Method according to claim 13, further comprising a step e) of washing the material obtained in step d) with distilled water.

15. Method according to claim 13 or 14, further comprising a step f) wherein the material is dried.

16. Method according to any one of claims 13-15, wherein the amount of the said pharmaceutical active principle bound to the said material depends from the time of contact with the solution of the said active principle and from the concentration of the said solution of active principle, according to predefined adsorption kinetic laws.

17. Material according to any one of the claims 1 - 12 for medical use.

18. Material according to claim 17 for antibiotic use.

## Patentansprüche

1. Polysaccharid oder Kohlenwasserstoff enthaltendes Material, das mit einem Monomer funktionalisiert ist, ausgewählt aus der Gruppe gebildet durch Glycidylmethacrylat oder dem Glycerinderivat davon, Acrylsäure, N-Vinylpyrrolidon, Acrylamid und Vinylacetat, **dadurch gekennzeichnet, dass** das Material mit mindestens einem pharmazeutischen Wirkstoff imprägniert ist und dass Cyclodextrine abwesend sind.

2. Material nach Anspruch 1, das ausgewählt ist aus einem Cellulosegewebe, einem Cellulosevlies, Polyamiden, Polyestern, Polypropylen oder anderen Polyolefinen oder Seide.

3. Material nach Anspruch 2, worin dieses, wenn es ein Cellulosegewebe oder ein Cellulosevlies ist, Leinen oder Baumwolle ist.

4. Material nach einem der Ansprüche 1-3, das ein Leinen- oder Baumwolltuch ist.

5. Material nach einem der Ansprüche 1-3, das ein Leinengegenstand, ein Kissenbezug, eine Bandage, eine Gaze, eine Gazeauflage für Operationen oder eine chirurgische Naht ist.

6. Material nach einem der Ansprüche 1-5, worin das Monomer ein Diolderivat von Glycidylmethacrylat ist.

7. Material nach einem der Ansprüche 1-6, das ein (3-Glycerin-O-carbonyl)-2-propyl funktionalisiertes Substrat ist.

8. Material nach einem der Ansprüche 1-7, worin der mindestens eine Wirkstoff ausgewählt ist aus der Gruppe bestehend aus antiinflammatorischen Arzneimitteln, Sedativa, Hypnotika, Analgetika, Anästhetika, Antibiotika, Arzneimittel für Herzerkrankungen, Antipyretika, Antidepressiva und Antispastika.

9. Material nach einem der Ansprüche 1-8, worin der mindestens eine Wirkstoff mindestens ein Antibiotikum ist, ausgewählt aus der Gruppe gebildet aus Penicillin, Aminoglycosiden, Chinolon-Antibiotika, Tetracyclin, Chloramphenicol, Sulfacetamid, Sulfamethazin, Sulfadiazin, Sulfamerazin, Sulfamethizol, Erythromycin, Clarythromycin, Rifamycin, Rifampycin, Ceftazidim, Amoxicillin, Amoxicillin-Trihydrat, Clavulansäure, Vancomycin-Chlorhydrat, Teicoplanin, Linezolid.

10. Material nach Anspruch 9, worin das mindestens eine Antibiotikum ausgewählt ist aus der Gruppe gebildet aus Rifamycin-Natriumsalz, Ceftazidim-Pentahydrat, Amoxicillin-Trihydrat und Kaliumclavulanat, Vancomycin-Chlorhydrat.

11. Material nach einem der Ansprüche 1-10, worin der mindestens eine Wirkstoff gegebenenfalls mit pharmazeutisch annehmbaren Hilfsstoffen formuliert ist.

12. Material nach einem der Ansprüche 1-11, worin die Menge des mindestens einen auf dem Cellulosesubstrat absorbierten Wirkstoffs im Bereich von 0,0001 % -60 % variiert.

13. Verfahren zur Herstellung des Materials nach einem der Ansprüche 1-12, umfassend die Schritte:
a) Bestimmen der Bildung von stabilen freien Radikalen mittels chemischer oder physikalischer Behandlung eines Cellulosesubstrats;
b) Reagieren der so gebildeten freien Radikale mit einem Monomer ausgewählt aus der Gruppe gebildet aus Glycidylmethacrylat, Acrylsäure, N-Vinylpyrrolidon, Acrylamid und Vinylacetat;
c) sofern das Monomer Glycidylmethacrylat ist, gegebenenfalls Behandeln des gemäß Schritt b) funktionalisierten Substrats in stark ionischen wässrigen Bedingungen, um so eine Öffnung des Expoxyrings zu bewirken, um das entsprechende Diolderivat zu ergeben;
d) Eintauchen des derivatisierten Substrats in eine wässrige Lösung von mindestens einem Wirkstoff für eine Zeit im Bereich von 1-96 Stunden.

14. Verfahren nach Anspruch 13, weiterhin umfassend einen Schritt e), wobei das in Schritt d) erhaltene Material mit destilliertem Wasser gewaschen wird.

15. Verfahren nach Anspruch 13 oder 14, weiterhin umfassend einen Schritt f), wobei das Material getrocknet wird.

16. Verfahren nach einem der Ansprüche 13-15, worin die Menge des an das Material gebundenen pharmazeutischen Wirkstoffs von der Kontaktzeit mit der Lösung des Wirkstoffs und der Konzentration der Lösung des Wirkstoffs entsprechend vordefinierter adsorptionskinetischer Gesetze abhängt.

17. Material nach einem der Ansprüche 1-12 zur medizinischen Anwendung.

18. Material nach Anspruch 17 zur antibiotischen Anwendung.

## Revendications

1. Matière porteuse de polysaccharides ou d'hydrocarbures fonctionnalisée avec un monomère sélectionné dans le groupe constitué par le méthacrylate de glycidyle ou le dérivé glycérol de ce denier, l'acide acrylique, le N-vinylpyrrolidone, l'acrylamide et l'acétate de vinyle, **caractérisé en ce que** ladite matière est imprégnés d'au moins un principe actif pharmaceutique et ne contient pas de cyclodextrine.

2. Matière selon la revendication 1, sélectionnée parmi un tissu à base de cellulose, un tissu non-tissé à base de cellulose, des polyamides, des polyesters, du polypropylène ou d'autres polyoléfines, ou de la soie.

3. Matière selon la revendication 2, la matière étant du lin ou du coton si elle est un tissu à base de cellulose ou un tissu non-tissé à base de cellulose.

4. Matière selon une des revendications 1 à 3, la matière étant une feuille de lin ou de coton.

5. Matière selon une des revendications 1 à 3, la matière étant un article en lin, une taie d'oreiller, un pansement, une gaze, une compresse de gaze destinée à être utilisée en chirurgie ou une suture.

6. Matière selon une des revendications 1 à 5, ledit monomère étant un dérivé diol de méthacrylate de glycidyle.

7. Matière selon une des revendications 1 à 6, la matière étant un substrat fonctionnalisé (3-glycérol-O-carbonyle)-2-propyle.

8. Matière selon une des revendications 1 à 7, ledit ou lesdits principes actifs étant sélectionnés dans le groupe constitué par les médicaments anti-inflammatoires, les sédatifs, les hypnotiques, les analgésiques, les anesthésiques, les antibiotiques, les médicaments destinés aux pathologies cardiaques, les antipyrétiques, les antidépresseurs, les antispastiques.

9. Matière selon une des revendications 1 à 8, ledit ou lesdits principes actifs étant au moins un antibiotique sélectionné dans le groupe constitué par la pénicilline, les aminoglycosides, les antibiotiques quinolones, la tétracycline, le chloramphénicol, la sulfacétamide, la sulfaméthazine, la sulfadiazine, la sulfamérazine, le sulfaméthizole, l'érythromycine, la clarythromycine, la rifamycine, la rifampicine, la ceftazidime, l'amoxicilline, le trihydrate d'amoxicilline, l'acide clavulanique, le chlorhydrate de vancomycine, la teicoplanine, la linézolide.

10. Matière selon la revendication 9, ledit ou lesdits antibiotiques étant sélectionnés dans le groupe constitué par le sel de sodium de rifamycine, le pentahydrate de ceftazidime, le trihydrate d'amoxicilline et le clavulanate de potassium, le chlorohydrate de vancomycine.

11. Matière selon une des revendications 1 à 10, ledit ou lesdits principes actifs pouvant être formulés avec des excipients pharmaceutiquement acceptables.

12. Matière selon un des revendications 1 à 11, la quantité dudit ou desdits principes actifs absorbée sur le substrat cellulosique variant de 0,0001 % à 60 %.

13. Procédé de préparation de la matière selon une des revendications 1 à 12, comprenant les étapes suivantes :
a) détermination de la formation de radicaux libres stables au moyen d'un traitement chimique ou physique d'un substrat cellulosique ;
b) mise en réaction des radicaux libres avec un monomère sélectionné dans le groupe constitué par le méthacrylate de glycidyle, l'acide acrylique, le N-vinylpyrrolidone, l'acrylamide et l'acétate de vinyle ;
c) si le monomère est du méthacrylate de glycidyle, traitement optionnel du substrat fonctionnalisé selon l'étape b) dans des conditions aqueuses fortement ioniques, de manière à provoquer l'ouverture du cycle époxy pour obtenir le dérivé diol correspondant ;
d) immersion du substrat dérivé dans une solution aqueuse d'au moins un principe actif pour une durée comprise entre 1 et 96 heures.

14. Procédé selon la revendication 13, comprenant en outre une étape e) de lavage à l'eau distillée de la matière obtenue à l'étape d).

15. Procédé selon la revendication 13 ou 14, comprenant une étape f) consistant à sécher la matière.

16. Procédé selon une des revendications 13 à 15, la quantité dudit principe actif pharmaceutique liée à ladite matière dépendant de la durée de contact avec la solution dudit principe actif et de la concentration de ladite solution de principe actif, selon des règles cinétiques d'adsorption prédéfinies.

17. Matière selon une des revendications 1 à 12, destinée à un usage médical.

18. Matière selon la revendication 17 destinée à un usage antibiotique.
